# EUROPEAN PATENT SPECIFICATION

(11) **EP 2 648 618 B1**
(45) Date of publication and mention of the grant of the patent: **14.07.2021**
(21) Application number: 11846913.9
(22) Date of filing: 09.12.2011
(51) Int. Cl.: A61B 5/18, A61B 5/02, A61B 5/08, G06K 9/00

(54) **SYSTEM FOR MONITORING A VEHICLE DRIVER**
SYSTEM ZUR ÜBERWACHUNG EINES FAHRZEUGFÜHRERS
SYSTÈME DE SURVEILLANCE D'UN CONDUCTEUR DE VÉHICULE

(30) Priority: 10.12.2010 US 422116 P
(43) Date of publication of application: 16.10.2013
(73) Proprietor: Joyson Safety Systems Acquisition LLC, Auburn Hills, MI 48326 (US)
(72) Inventor: WATSON, William Todd, Belleville, Michigan 48111 (US); MURESAN, Adrian Valentin, Canton, Michigan 48187 (US); KRAUSE, Joseph Kae, Northville, Michigan 48168 (US); TANG, Yipeng, Troy, Michigan 48084 (US)
(74) Representative: J A Kemp LLP
(86) International application number: PCT/US2011/064185
(87) International publication number: WO 2012/078996

(56) References cited:
- EP-A1- 1 452 127
- WO-A1-02/50792
- WO-A1-99/36893
- WO-A1-2005/120878
- US-A1- 2001 022 550
- US-A1- 2008 101 659
- US-A1- 2009 058 660
- US-A1- 2009 231 145
- US-A1- 2009 261 979
- US-A1- 2009 261 979
- US-A1- 2010 234 741
- US-B1- 7 027 621
- US-B2- 6 856 873
- US-B2- 7 777 778
- MING-ZHER POH ET AL: "Non-contact, automated cardiac pulse measurements using video imaging and blind source separation", OPTICS EXPRESS, vol. 18, no. 10, 10 May 2010 (2010-05-10), page 10762, XP055016649, ISSN: 1094-4087, DOI: 10.1364/OE.18.010762

## Description

### CROSS-REFERENCE TO RELATED APPLICATIONS

This application claims priority to and the benefit of U.S. Provisional Patent Application No. 61/422,116 filed December 10, 2010.

### BACKGROUND

The present disclosure relates generally to the field of biological and physiological signal monitoring. Biological or physiological parameters, measures or data are generated by biological activity of a person's body and may include, for example, heart rate or breathing rate. The biological measures may be used to monitor a person and determine if the person is alert. For example, in a vehicle, the biological measures may be used to determine the driver's performance and maintain the performance within an optimal range. Biological measures may indicate driver stress, stimulation, fatigue, or relaxation, which can increase the likelihood that the driver may make an error, experience a reduction in safe driving performance, etc.

Current camera-based systems are capable of performing optical monitoring of a human's face, for example, to obtain biological or physiological signals. However, optical monitoring may be more difficult or inaccurate in low ambient light conditions, such as in vehicles during certain driving conditions. Certain driving conditions reduce the amount of ambient light in the vehicle leading to a smaller signal-to-noise ratio (SNR) of the biological measures. What is needed are systems and methods to allow a camera-based system located in a vehicle, along with other vehicle systems such as lighting systems and various sensors, to obtain the accurate biological measures needed to monitor the driver's performance using optical techniques. WO 99/36893 A describes that, in a process of detecting a person falling asleep, an image of the face of the person is acquired. Pixels of the image having characteristics corresponding to an eye of the person are selected and a histogram is formed of the selected pixels. The histogram is analyzed over time to identify each opening and closing of the eye, and characteristics indicative of the person falling asleep are determined. A sub-area of the image including the eye may be determined by identifying the head or a facial characteristic of the person, and then identifying the sub-area using an anthropomorphic model. To determine openings and closings of the eyes, histograms of shadowed pixels of the eye are analyzed to determine the width and height of the shadowing, or histograms of movement corresponding to blinking are analyzed. An apparatus for detecting a person falling asleep includes a sensor for acquiring an image of the face of the person, a controller, and a histogram formation unit for forming a histogram on pixels having selected characteristics. Also disclosed is a rear-view mirror assembly incorporating the apparatus. WO 02/50792 A describes a high reliable safety system intended for preventing catastrophes (accidents) of technic-technological system man-machine that would be caused due to drowsiness of a man. The highest degree of reliability (100%) with reference to the system security preservation has been founded upon the development of a new method for early detection and monitoring of a drowsiness process course of a man (Com. A. PIA DYNAMICS), by means of which the drowsiness process is detected at the moment of its commencement and continuously monitored up to the moment of the sleeping process occurrence. Early detection of drowsiness of a man creates real prerequisites for timely performance of procedures for the system security preservation such as: the procedure for adaptable system control with the purpose of permanently accommodating the speed of the vehicle to the driver awareness degree fall found out and the procedure for preventing the driver drowsiness (crisis biological factor) at the early drowsiness phase when the chances for success are maximum. The invention is controlled by the processing computer (14), so that both procedures for the crisis factors prevention are carried out in real time with finding out certain drowsiness process phases (out of six in total) simultaneously or in the "step-by-step" procedure. Failure of the driver drowsiness process prevention procedure in the third step of repetition (maximum dose of excitement) automatically is a sign that a hard form of drowsiness, that is, high degree of the system endangerment is in question, in which case the problem of further system security preservation is resolved by the invention by means of the adaptable system control procedure. Based on the results obtained from the vehicle speed decreased over the previous steps of action to the value of v = 20 km/h, the vehicle is timely stopped through the last step of action (v = 0 km/h) at the road length 1 = I m, practically while the driver is still in the state of consciousness (SB = 45%, 10-2 sec < tp.r.< 10-1 sec, t V.B. = 0). US 2001/022550 A describes a monitoring device for vehicles having a housing and at least one mirror glass arranged in the housing so as to have a front side facing an observer. The at least one mirror glass has a reflective layer being reflective in the visible spectral range of light. At least one camera is arranged behind the reflective layer in a viewing direction viewed from the front side. The monitoring device can be used for driver identification, monitoring the driver's condition, identifying passengers and passenger positions, controlling airbags, theft surveillance, and similar purposes. US 2009/231145 A describes an input apparatus for a vehicle including an operation element operable by an occupant of the vehicle; a biological information acquisition element acquiring biological information of the occupant; an unawakened state detection element detecting an unawakened state of the occupant based on the biological information, wherein the unawakened state is defined by a predetermined state different from an awakened state; and an operation disabling element disabling an operation input from the operation element when the unawakened state detection element detects the unawakened state. US 2009/261979A describes a method and system for monitoring a vehicle driver including an optical imaging system that obtains images of the driver, and a processor coupled to the optical imaging system and arranged to analyze the images obtained by the optical imaging system to locate a head of the driver in the images and monitor the driver's head or a part thereof over time. The processor also determines, based on the monitoring of the driver's head or part thereof, whether the driver has lost the ability to operate the vehicle. A reactive component is affected by the processor's determination that the driver has lost the ability to operate the vehicle, and requires action by the driver to indicate regaining of the ability to operate the vehicle or exerting control over the vehicle to slow the vehicle and bring it to a stop. US 7,027,621 B describes a system using passive infrared imaging of the face and other body parts of an operator to obtain observables by automatically extracting features from a sequence of images, analyzing the extracted features, and then assessing the results for indicators of performance of a task by the operator in order to provide early warning of potential cognitive or motor impairment and thereby facilitate risk reduction and quality maintenance. The infrared condition monitoring system (IR-CMS) serves to a) assess cognitive and/or physical readiness to perform a particular task; b) provide condition assessment feedback to the subject and his appropriate supervisors; c) activate measures to increase short-term alertness and other readiness factors; d) limit potential risks by restricting the subject's access, responsibility, or authority; and e) facilitate rapid medical treatment, evacuation, quarantine, re-training, or counseling as appropriate. The same condition monitoring and assessment system can also be used during training and simulator exercises to evaluate personnel for assignment. US 2010/0234741A discloses a drowsiness detection method. A heartbeat signal and a breathing signal are detected by exploiting together a scheme and an optical system scheme. The detected signals are applied to respective amplification units, noise signals are eliminated from the detected signals, and noise-free signals are amplified. The amplified signals are applied to a central processing unit, signal processing is processed on the signals, and processed signals are combined. The combined signal is counted, and a warning sound, voice message or vibration is output in a case where a value, obtained by subtracting a counted output value monitored one minute before a current time, from a counted output value monitored two minutes before the current time, falls within a detection range and where, with a passage of time, the value falling within the detection range is successively detected from two to ten times. EP 1 452 127 A describes an apparatus for pupil detection. First light is emitted from a first light source at a first illumination angle relative to the axis of a detector. Second light is emitted from a second light source at a second illumination angle relative to the axis. The first light and the second light can have substantially equal intensities. The second illumination angle is greater than the first illumination angle. Reflected first light and reflected second light are received at the detector. The difference between the reflected first light and the reflected second light can be determined. The difference can be used to detect the pupils of a subject's eyes. WO 2005/120878 A describes a security device for a means of transport comprising a radiation source to direct radiation into the eye of a person intending to take control of the means of transport, so that the radiation is internally reflected within the eye and emerges from the eye with a substantial spectral content corresponding to the spectral content of food flowing through the retina of the eye, and a receiver arrangement to receive radiation emanating from the eye of the person and to perform a spectral analysis of the reflected radiation to determine the concentration of at least one chemical within the blood of the person, the device further comprising an alarm unit to generate an alarm and/or an immobiliser unit to immobilise the means of transport, the or each unit being responsive to the said determination of concentration when the concentration of the said chemical is outside a permitted threshold range. Preferably, radiation is transmitted to the retina, and the spectral analysis is carried out for radiation reflected from the retina. US 2009/0058660 A describes a biosensor, communicator, and/or controller apparatus, systems, and methods for monitoring movement of a person's eye. The apparatus includes a device configured to be worn on a user's head, a light source for directing light towards one or both eyes of the user, one or more image guides on the device for viewing one or both eyes of the user, and one or more cameras carried on the device and coupled to the image guides for acquiring images of the eyes and/or the user's surroundings. The apparatus may include a cable and/or a transmitter for transmitting image data from the camera to a remote location, e.g., to processor and/or display for analyzing and/or displaying the image data. A system including the apparatus may be used to monitor one or more oculometric parameters, e.g., pupillary response, and/or to control a computer using the user's eyes instead of a mouse. MING-ZHER POH ET AL: "Non-contact, automated cardiac pulse measurements using video imaging and blind source separation", OPTICS EXPRESS, vol. 19, no 10, 10 May 2010 describes that remote measurements of the cardiac pulse can provide comfortable physiological assessment without electrodes. However, attempts so far are non-automated, susceptible to motion artifacts and typically expensive. This paper introduces a new methodology that overcomes these problems. This novel approach can be applied to color video recordings of the human face and is based on automatic face tracking along with blind source separation of the color channels into independent components. Using Bland-Altman and correlation analysis, the paper compares the cardiac pulse rate extracted from videos recorded by a basic webcam to an FDA-approved finger blood volume pulse (BVP) sensor and achieved high accuracy and correlation even in the presence of movement artifacts. Furthermore, it applies this technique to perform heart rate measurements from three participants simultaneously.

### SUMMARY

According to the present invention, there is provided a system as defined in appended claim 1.

### BRIEF DESCRIPTION OF THE DRAWINGS

These and other features, aspects, and advantages of the present invention will become apparent from the following description, and the accompanying exemplary embodiments shown in the drawings, which are briefly described below.
FIGS. 1A-B are perspective views of a vehicle and vehicle interior where a controller for a driver monitoring system may be used.
FIG. 2 is a block diagram of a system controller connected to various vehicle systems.
FIG. 3 is a block diagram of a controller used in a system for monitoring the vehicle driver.
FIG. 4 is a flow chart of a process for adjusting a lighting level for a camera used in a system for monitoring a vehicle driver.
FIG. 5 is a block diagram of a system controller used in a system for monitoring a vehicle driver.
FIG. 6 is a block diagram of a controller for a vehicle driver monitoring system.
FIG. 7 is a flow chart of a process for using sensor and transducer signals to improve the quality of a biological measure.

### DETAILED DESCRIPTION

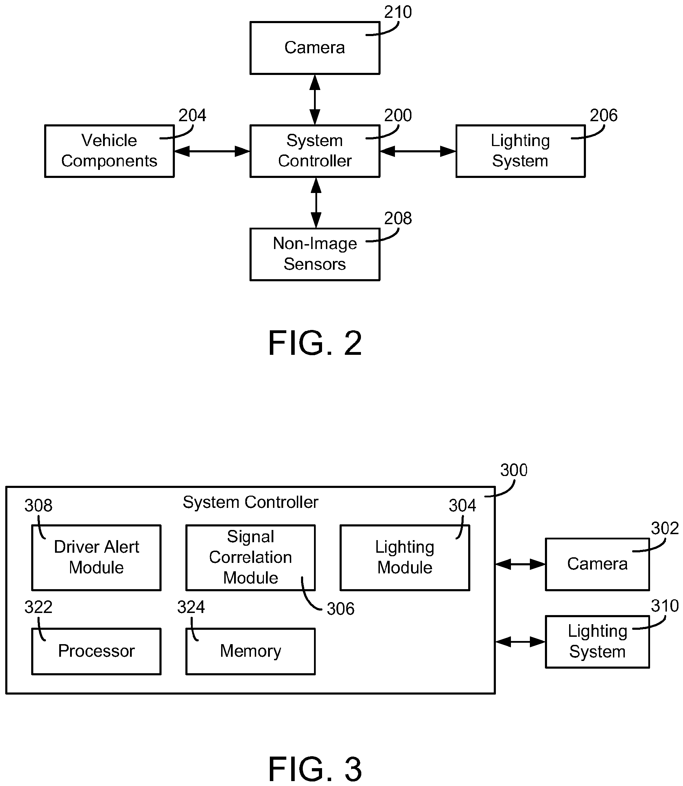

Generally described herein are figures, systems and methods for using a system controller to monitor biological measures of a vehicle driver. Referring to FIG. 2, a system controller 200 is coupled to a camera or multiple cameras 210 for obtaining biological or physiological signals such as heart rate and breathing rate, for example. According to one exemplary embodiment, a camera or multiple cameras in conjunction with a system controller may obtain biological or physiological information about an occupant of a vehicle in a manner as described in U.S. Patent App. No. 13/048,965 filed March 16, 2011, entitled "Method and System for Measurement of Physiological Parameters". Once a biological or physiological parameters are obtained, the system controller 200 may then determine if the driver 12 is in an optimal state for operating the vehicle.

The system controller 200 and camera 210 includes or is coupled to a lighting system 206 for adjusting the lighting level of a portion of the driver's body 12. The lighting system 206 may include the lighting system of the vehicle with light sources positioned throughout the vehicle. The lighting system may also include lights associated with the camera 210 coupled to the system controller 200 or supplemental light sources other than the vehicle lighting system. For example, if the ambient light level is low in the vehicle, the system controller 200 may determine that additional lighting should be provided and the lighting system may be configured to provide additional light such that the camera 210 may more accurately obtain the biological measures for the system controller. The system controller is coupled or operatively connected to additional sensors 208 such as, infrared sensors, light sensors, as well as non-image sensors, such as pressure sensors, for example. The camera 210 may include an infrared detector and multiple frequency detection capabilities through the use of an optical filter.

The additional sensors 208 may be used to measure the biological condition of the driver, and the system controller 200 may use inputs from both the non-image sensors 208 and the camera 210 to more accurately measure the biological condition when image data from the camera 210 is of a poor quality. In addition some of the signals from non-image sensors 208 may have varying degrees of reliability. For example, some signals may have low SNR because of signal disturbances such as vibration and proximity to the person being sensed, for example. Therefore, the system controller 200 may receive both image signals from a camera 210 and non-image signals from additional sensing units 208 and correlate the signals through blind source separation or other correlation techniques. Further, the system controller 200 and camera 210 may be configured to only measure specific frequencies that contain stronger biological information of interest through optical filtering techniques. Additionally, filtering the light detected by the camera 210 may reduce or remove the noise present in the signal obtained by the camera 210. It should be understood that any combination of systems described herein may be used by the system controller 200 to obtain and use the biological measures.

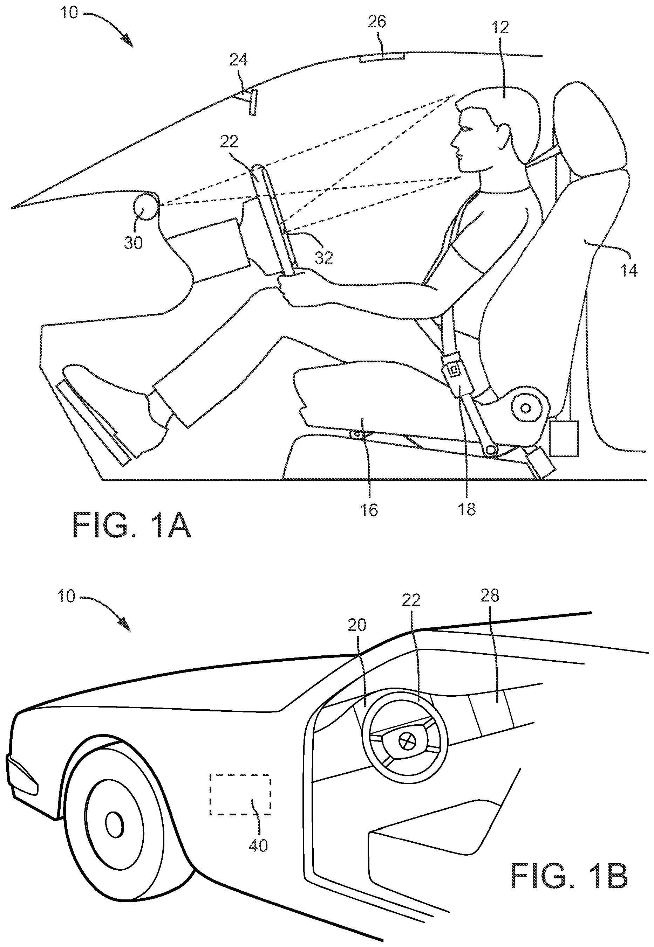

Referring now to FIGS. 1A-B, perspective views of a vehicle 10 and a driver 12 are shown. The vehicle 10 may include a system controller 40 coupled to a camera 30 and/or 32. The camera 30 and/or 32 may be located on or near the instrument panel display 20 of the vehicle 10, in the steering wheel 22 of the vehicle 10, or in any other location of the vehicle 10 such that the camera 30 and/or 32 has a clear view of the driver 12's face and/or body so that the camera 30 and/or 32 is capable of detecting image data of the vehicle driver. The camera 30 and/or 32 may provide the system controller 40 with signals containing consecutive frames of image data. This image data can then be analyzed by system controller 40 to obtain biological paramters, such as a heart rate or a respiration rate, derived from sensed biological measures of the driver 12.

The vehicle 10 may further include light sources that may provide additional lighting for the system controller. The additional lighting may allow for the camera 30 and/or 32, for example, to more easily obtain biological measures from the driver 12. The light sources may be incorporated into the instrument panel display 20, the steering wheel 22, the rearview mirror 24, button lighting 26 for the vehicle heads up display, a center console 28 of the vehicle, or in another location.

The vehicle 10 may further include sensors or other devices located in vehicle 10 configured to obtain biological measures of the driver, such as non-image sensors 208. The non-image sensors 208 and other devices may include piezoelectric sensors, pressure sensors, ultrasonic transducers, electric field transducers, infrared sensors, or microphones, for example. The biological measures obtained by the non-image sensors 208 may be combined with the biological measures obtained by the camera 30 and/or 32 to improve the accuracy of the determined biological paramters. Non-image sensors may be located in the seat back 14, seat bottom 16 (e.g., pressure sensors), seat belt 18 (e.g., pressure sensors, piezoelectric sensors), steering wheel 22 of the (e.g., an infrared sensor or light sensor pointing towards the driver 12), or elsewhere in the vehicle 10, for example. A system controller 40 may be in the vehicle 10 and may be coupled to the various cameras 30 and/or 32, non-image sensors 208, or various other vehicle components. The system controller 40 may transmit signals to the various vehicle components and provide a warning, alert, or other output to vehicle components based on the received signals.

Referring now to FIG. 2, a block diagram of a system controller 200 and various vehicle systems are shown, including a vehicle components 204. Vehicle components 204 may include a display, a vehicle electronic control unit (ECU), a lighting system, a breaking system, and a microphone or sound system, for example. The system controller 200 may provide an output signal to one or several vehicle components 204 where the signal carries instructions relating to vehicle operation. For example, the system controller 200 may provide an output signal that prompts a the display with information to display data on an instrument panel display, center console of the vehicle, or a HUD of the vehicle. The output signal may contain information including a warning or alert that the driver 12 is not driving optimally or may not be in condition to drive based on a condition signal derived from image data of the driver 12 received from camera 210.

The system controller 200 may provide an output signal to the vehicle ECU relating to the operation of the vehicle. The system controller 200 may provide an output signal comprising instructions to the vehicle ECU. For example, the system controller 200 may instruct the vehicle ECU 204 to stop the vehicle if the system controller 200 determines a condition of the driver is prohibiting the driver from properly operating the vehicle. The system controller 200 may provide an output signal that causes one or multiple vehicle components 204 to provide suggestions or warnings to the driver 12. Additionally, system controller 200 may transmit an output signal that causes or directs altering of the operation of the vehicle 10.

The system controller 200 may provide information to the lighting system 206 of the vehicle. The lighting system 206 may adjust the lighting inside the vehicle as a result of a command from the system controller 200. For example, if the system controller 200 determines that a camera 210 needs more light, the intensity of light from the light sources of lighting system 206 may be increased. The direction of the light source may also be adjusted to illuminate (or dim) the appropriate portion of the driver's body. The system controller 200 may have a predetermined threshold stored in memory to determine if the ambient light detected in the vehicle is sufficient for producing image data of the driver 12. The system controller 200 may provide information to a microphone or sound system 208 of the vehicle. The sound system 208 may provide an audible warning of the condition of the driver to the driver or other occupants of the vehicle. The system controller 200 is further coupled to a camera 210 for obtaining biological measures. The system controller 200 may further be coupled to vehicle components 204 relating to the operation of the vehicle, and may provide output signals to those components 204 based on a condition signal associated with a biological or physiological condition of a vehicle driver based on the image data. As an example, the condition signal may be a measure of the heart rate or the breathing rate of the vehicle occupant 12.

Referring now to FIG. 3, a system controller 300 useful for an understanding of the invention is shown in greater detail. The system controller 300 includes a camera 302, a lighting module 304, a signal correlation module 306, a driver alert module 308, at least one processor 322, and at least one memory 324. The controller 300 is used in a system for monitoring a vehicle driver such as shown in Fig. 2, for example. The various modules may comprise software instructions stored in memory 324 and executed by processor 322. For example, the lighting module 304 may receive an input from the camera 302 and determine a change in lighting levels. Based on this determination, lighting module 304 of system controller 300 may send an output signal to lighting system 310 to increase the intensity of one or more light sources, or to turn on additional light sources. Alternatively, lighting module 304 may determine that there is sufficient ambient light available in the vehicle such that the camera 302 can detect the biological measures from the image data captured at camera 302.

The lighting module 304 may determine and/or set the light frequency of the additional light provided. Some light frequencies allow for greater detection of biological measures than other light frequencies. In particular, detection of a cardiovascular pulse wave through plethysmographics techniques by the camera 302 may be enhanced when using frequencies corresponding to yellow/green visible light at about 520-585 nanometers (nm). However, projecting significant light at this frequency on the face of the driver may impede the driver in low ambient light conditions. Hemoglobin is known to have a higher absorption at frequencies around 400 nm, the transition from visible to ultraviolet light, where the human eye is less sensitive to light. Therefore, a supplemental light source provided at this frequency is more desirable as the light is less noticeable by the driver. Light sources may be incorporated in various locations within the vehicle such as the instrument panel display, center stack display, steering wheel lighting, button lighting, or on a camera 302. The light frequencies may be pulsed at a reduced duty cycle such that camera 302 shutter may be synchronized with the pulses. In such cases, a lower overall light intensity is projected onto the driver's face while keeping the SNR of image data detected at the camera 302 high.

Furthermore, face and eye tracking performed by the camera 302 may be used to collimate and/or direct a supplemental light source within lighting system 310 towards the driver's face in a direction or pattern to reduce projection of visible light into the eyes of the driver. The lighting system 310 and the camera 302 may also be directed towards the driver's hands or other exposed skin in order to avoid directing the light towards the driver's eyes.

The signal correlation module 306 receives a signal from the camera 302 and signal is configured to analyze the signals. For example, the signal correlation module 306 may receive the signals and use filtering to separate noise from the signal.

The system controller 300 further includes a driver alert module 308. Driver alert module 308 may receive and analyze data from the lighting module 304 and/or signal correlation module 306. The driver alert module 308 may determine, based on the heart rate, breathing rate, or another metric of the biological state of the driver, that the driver is incapable or at risk of not operating the vehicle properly. The driver alert module 306 may provide an output signal containing a message to a vehicle component 204 or a haptic warning device relating to the determination.

The processor 322 and memory 324 may be used to carry out processes described herein. The processor 322 may be a general purpose processor, an ASIC, or another suitable processor configured to execute computer code or instructions stored in memory 324. The memory 324 may be hard disk memory, flash memory, network storage, RAM, ROM, a combination of computer-readable media, or any other suitable memory for storing software objects and/or computer instructions. When the processor 322 executes instructions stored in memory 324 for completing the various activities described herein, the processor 322 generally configures system controller 300 to complete such activities. The processor 322 may be coupled to the various modules of the system controller 300 or include the various modules of the system 300, such as, for example, a driver alert module 306. The modules may be software or hardware and may be analog or digital. Each module may further include an additional processor similar to the processor 322. In such embodiments, the processor 322 may be omitted.

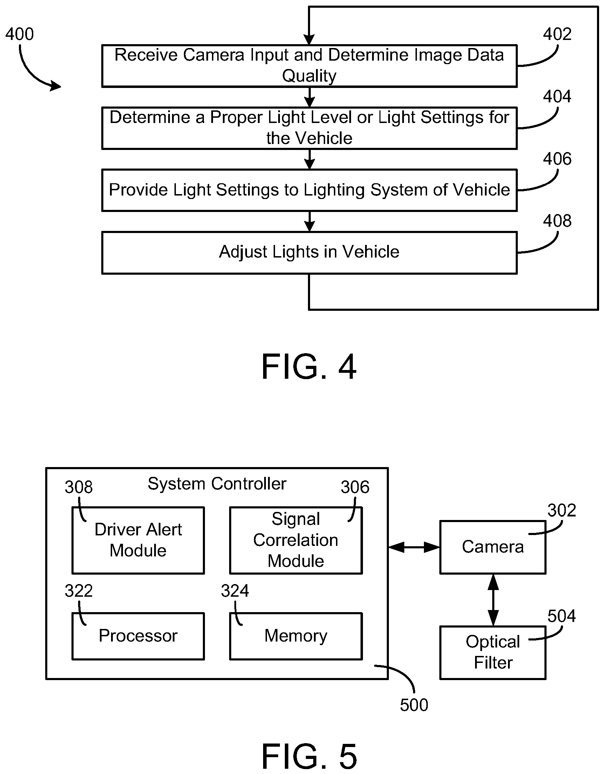

Referring also to FIG. 4, a flow chart of an exemplary process 400 for adjusting lighting levels using the system controller 300 is shown. After receiving image data of a vehicle driver from the camera 302 and determining if image quality, such as an ambient light level in the vehicle is above or below a predetermined threshold at step 402, the lighting module 304 may determine a proper lighting level or light settings at which the camera 302 can detect or view certain attributes that can measure the biological condition at step 404. Once a proper light setting has been determined at step 404, the light settings may be provided to a lighting system such as lighting system 206 at step 406. The lights of the vehicle are then adjusted at step 408 based on an output signal from the system controller 300, according to one embodiment. The process shown in FIG. 4 may be iterative or non-iterative.

Referring now to FIG. 5, another system controller 500 useful for an understanding of the invention is shown. The controller 500 is used in a system for monitoring a vehicle driver such as shown in Fig. 2, for example. The camera 302 is shown coupled to an optical filter 504 for filtering the light detected by the camera 302. Using the optical filter 504, the system controller 500 may enhance the SNR of the signals received from the camera 302 by only measuring frequencies that contain stronger biological measures of interest. For example, hemoglobin is known to absorb light more strongly in the yellow-green frequency range, approximately between 520-585 nm, and in the violet frequency range, approximately between 390-420 nm. Filtering the light, using optical filter 504, to only detect a portion of the yellow-green or violet frequency range will remove noise present in other adjacent frequency bands, improving the SNR. The improved signals may then be used by the signal correlation module 306 and driver alert module 308 to more accurately determine the condition of the driver. The system controller 500 further includes a processor, and memory as described in FIG. 3.

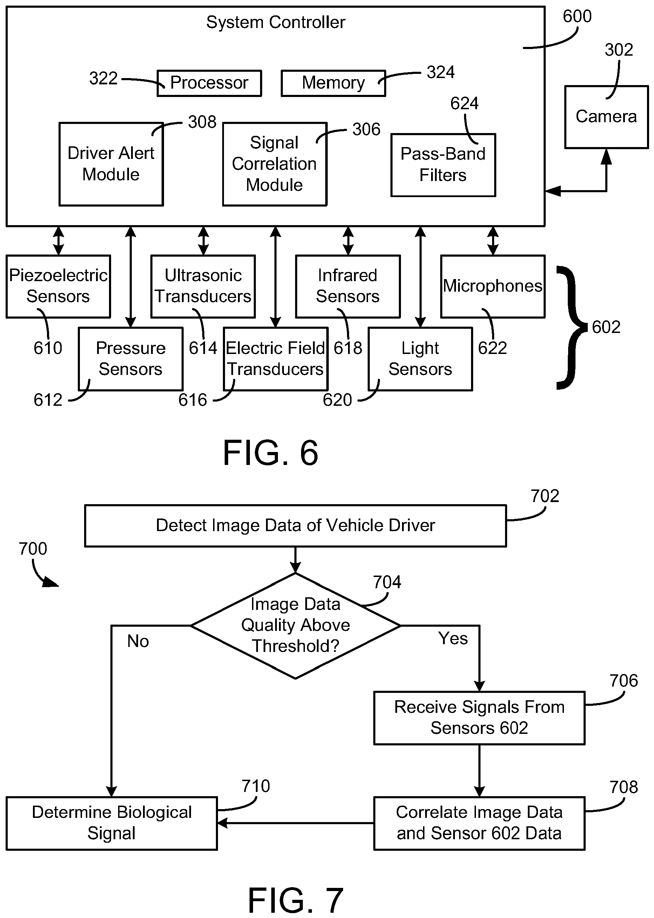

Referring now to FIG. 6, a system controller 600 is shown. The controller 600 is used in a system for monitoring a vehicle driver such as shown in Fig. 2, for example. In FIG. 6, various sensors and transducers 602 are shown coupled to the system controller 600 and may provide system 600 and the signal correlation module 306 with data regarding biological measures detected by the sensors and transducers 602. The signals from the sensors and transducers 602 may be correlated with the signals from the camera 302 to determine the biological parameters of the driver of the vehicle. The system controller 600 may use the signals from the sensors and transducers 602 when there is not enough ambient light for the camera 302. In the claimed invention, if the system controller 600 determines that the image data captured by camera 302 has a poor image quality, for example an image quality below a predetermined threshold stored in memory 324, such that biological parameters of the driver 12 cannot be derived from the image data, the system controller 600 alters the lighting system 206 or retrieves data from non-image sensors 208 to either improve the image data quality or supplement the image data with non-image sensor data to derive a condition signal related to biological condition of the driver 12.

The signals from the camera 302 may be correlated with the signals from the various sensors 602 such as non-image sensors that non-intrusively detect the biological measures of interest. Each individual biological measure detected by the sensors may have an inherent weaknesses such as a low SNR during mechanical vibration, low SNR under electric field bombardment, or low SNR when the distance from the sensor to the driver increases, for example. However, when the signals are correlated with low SNR signals from other sensors, transducers, and the camera 302 by signal correlation module 306, an improved indicator of the physiological condition for the driver may be derived by the signal correlation module 306 and provided to the driver alert module 308.

The sensors 602 may include one or more piezoelectric sensors 610. The piezoelectric sensors 610 may be located in the vehicle and coupled to the system controller 600. The piezoelectric sensors 610 may be located in the seat 14, 16, the seatbelt 18, or the steering wheel 22 of the vehicle 10 and may measure the dynamic pressure or force applied by the driver 12 of the vehicle 10 to the seat 14, 16, seatbelt 18, steering wheel 22, or another portion of the vehicle 10 the driver 12 is in contact with.

The sensors 602 may include one or more pressure sensors 612. The pressure sensors 612 may be located in the vehicle and be coupled to the system controller 600. The pressure or force sensors 612 may be located in the seat 14, 16, the seatbelt 18, or the steering wheel 22 of the vehicle. The pressure sensors 612 may include air/fluid bladders, pressure sensitive inks, force sensitive resistors, or strain gages, and provides the system controller 600 with a signal relating to the pressure applied by the driver 12 of the vehicle 10 to the seat 14, 16, seatbelt 18, steering wheel rim 22, or another portion of the vehicle 10 the driver 12 is in contact with.

The sensors 602 may include ultrasonic transducers 614. The ultrasonic transducers 614 may be located in the vehicle and provide the system controller 600 with signals relating to high frequency sound waves that have reflected from the driver's body and/or clothes. The ultrasonic transducers 614 may be located in the seatbelt 18, the instrument panel 20, the steering wheel 22, or another area of the vehicle 10. Capacitively coupled electric field transducers 616 may be located in the seat 14, 16, the seatbelt 18, the steering wheel 22, or another area of the vehicle 10 and provide the system controller 600 with signals regarding the electric field.

The sensors 602 may include infrared sensors 618. The infrared sensors 618 or photodiodes may be included in the vehicle 10, for example, in the dashboard 20 or steering wheel 22 and may be pointed towards the driver 12's face or hands. The infrared sensors 618 measure infrared light naturally radiating from the driver 12 or reflected from the driver 12 by a supplemental or natural infrared light source and provide the system controller 600 with signals relating to the measured dynamic infrared light intensity. More generally, light sensors 620 may also be included in the vehicle 10 (e.g., in the steering wheel or dashboard of the vehicle) and may be pointed towards the driver 12's face or hands. The light sensors 620 detect light and light changes and provides the system controller 600 with signals relating to the light changes. The light sensors 620 are supplemented by a mostly non-visible light source. Furthermore, light sensors 620 and infrared sensors 618, and any of the sensors 602 may be associated with lighting system 206, non-image sensors 208, vehicle components 204, or camera 210 depending on a desired implementation.

The sensors 602 may include microphones 622 for detecting sound and providing the system controller 600 with signals relating to sounds made by the driver 12. The microphones 622 may be located in the seat 14, 16, the seatbelt 18, the steering wheel 22, or otherwise.

The system controller 600 is shown to include one or more band-pass filter 624 for detecting multiple light frequencies. The band-pass filters 624 may be applied to the image signals provided by the camera 302 or subsets of pixels from the images from camera 302.

The signal correlation module 306 may receive the various signals from the sensors and transducers 602 and from the camera 302. The biological parameter of interest may be identified by the signal correlation module 306 using various correlation techniques. For example, blind source separation (BSS) may be used to separate and identify the relevant biological measures. For physiological parameters such as heart rate and breathing rate, some signal separation from noise may be achieved by band-pass filtering (e.g., by the band-pass filter 624), because an expected frequency range for the signals is known. However, there is frequency overlap from noise sources that cannot be removed by conventional hardware/software filtering. Thus, BSS techniques may be used including principal component analysis (PCA), independent component analysis (ICA), or sparse component analysis (SCA). The driver alert module 308 may use the signal from the signal correlation module 306 and determine if an alert or another change in the operation of the vehicle should be made in response to the current condition of the driver. After BSS techniques are applied, Fast Fourier Transform (FFT) techniques may be applied to process the signals further to extract frequency based biometrics such as breathing rate and respiration rate.

Referring also to FIG. 7, a flow chart of a process 700 for using signals from additional sensors 602 such as non-image sensors 208 to improve the quality of a biological measure is shown. The system controller 600 may receive image data from a camera 302 and determine if the quality of the image data is sufficient to derive a biological parameter of the vehicle driver 12 at step 704. The quality of the image data may be based on one or more of SNR, ambient light detected, or signal strength, for example. If the image data is sufficient to produce a reliable biological parameter of the driver 12, the process determines at least one biological parameter of the vehicle driver 12 at step 710. However, if the image data from camera 302 is insufficient, for example, by failing to meet or surpass a predetermined image quality threshold, system controller 600 requests signals from one or more of the sensors 602, such as non-image sensors at step 706. Once additional signals from sensors 602 are received at step 706, process 700 correlates the image and non-image data at the signal correlation module 306, for example, to determine a reliable biological parameter at step 710. As stated previously, the determined biological parameter may then be used to by the driver alert module 308 to send an output signal to a vehicle component 204 in order to alert the driver of a unsuitable condition. The biological parameter may be respiration rate and the output signal prompts an speaker to emit an audible command for the purpose of waking the driver up if the respiration rate is too low.

Although the system controller is illustrated as including multiple features utilized in conjunction with one another, the system may alternatively utilize more or less than all of the noted mechanisms or features. For example, in other exemplary embodiments, there may be more or fewer than the illustrated sensors, various modules of the embodiments of FIGS. 3, 5 and 6 may be combined, etc. Further, the system controller may be used in an environment other than a vehicle.

Although specific shapes and locations of each element have been set forth in the drawings, each element may be of any other shape or location that facilitates the function to be performed by that element. For example, the cameras and light sources have been shown in particular vehicle locations; however, in other exemplary embodiments the sensing elements may be located anywhere in the vehicle.

For purposes of this disclosure, the term "coupled" means the joining of two components (electrical, mechanical, or magnetic) directly or indirectly to one another. Such joining may be stationary in nature or movable in nature. Such joining may be achieved with the two components (electrical or mechanical) and any additional intermediate members being integrally defined as a single unitary body with one another or with the two components or the two components and any additional member being attached to one another. Such joining may be permanent in nature or alternatively may be removable or releasable in nature.

The present disclosure has been described with reference to example embodiments, however persons skilled in the art will recognize that changes may be made in form and detail without departing from the scope of the invention defined by the appended claims.

Exemplary embodiments may include program products comprising computer or machine-readable media for carrying or having machine-executable instructions or data structures stored thereon. For example, the system controller may be computer driven. Exemplary embodiments illustrated in the methods of the figures may be controlled by program products comprising computer or machine-readable media for carrying or having machine-executable instructions or data structures stored thereon. Such computer or machine-readable media can be any available media which can be accessed by a general purpose or special purpose computer or other machine with a processor. By way of example, such computer or machine-readable media can comprise RAM, ROM, EPROM, EEPROM, CD-ROM or other optical disk storage, magnetic disk storage or other magnetic storage devices, or any other medium which can be used to carry or store desired program code in the form of machine-executable instructions or data structures and which can be accessed by a general purpose or special purpose computer or other machine with a processor. Combinations of the above are also included within the scope of computer or machine-readable media. Computer or machine-executable instructions comprise, for example, instructions and data which cause a general purpose computer, special purpose computer, or special purpose processing machines to perform a certain function or group of functions. Software implementations of the present invention could be accomplished with standard programming techniques with rule based logic and other logic to accomplish the various connection steps, processing steps, comparison steps and decision steps.

It is also important to note that the construction and arrangement of the elements of the system as shown in the preferred and other exemplary embodiments is illustrative only.

## Claims

1. A system configured to receive and improve the quality of image data used to determine the condition of a vehicle driver (12), the condition of the vehicle driver (12) being determined by deriving from the image data a condition signal associated with a biological or physiological condition of the vehicle driver (12) related to biological measures including at least one of the heart rate of the vehicle driver (12) and the breathing rate of the vehicle driver (12), the system comprising:
a camera (302) positioned to detect image data of the vehicle driver (12);
non-image sensors (602) for providing non-image sensor data regarding biological measures of the vehicle driver (12);
a lighting system (206, 310) positioned to illuminate an interior of the vehicle;
a controller (600) configured to receive the detected image data and to receive the non-image sensor data from the non-image sensors (602); wherein:
the controller (600) is configured to determine the condition of the vehicle driver (12) based on the received image data;
the controller (600) is configured to make a determination of the quality of the image data; and
the controller is configured to, if the controller (600) determines that the received image data has a poor image quality such that the biological measures of the vehicle driver (12) cannot be derived from the received image data, selectively either alter the lighting system based on the determined image data quality to improve image data quality or determine the condition of the vehicle driver (12) based on both the image data of the vehicle driver (12) and the non-image sensor data from the non-image sensors (602) by supplementing the image data with the non-image sensor data.

2. The system of claim 1, wherein the controller (600) is further configured to send an output signal to a vehicle component based on the determined condition of the vehicle driver (12).

3. The system of claim 2, wherein the output signal prompts one of: display data to be presented on a display device located in the vehicle interior; and
audible data to be emitted from a sound system.

4. The system of claim 1, wherein altering the vehicle lighting system (206, 310) comprises increasing the intensity of at least one light source in the lighting system (206, 310).

5. The system of claim 1, wherein altering the vehicle lighting system (206, 310) comprises adjusting the frequency of the image data detected at the camera (302).

6. The system of claim 1, wherein the non-image sensors (602) include at least one of a pressure sensor, an electric field transducer, a piezoelectric sensor, an ultrasonic transducer, and a microphone.

7. The system of claim 1, wherein the image data quality is a measure of signal to noise ratio.

## Patentansprüche

1. System, das dafür konfiguriert ist, die Qualität von Bilddaten zu empfangen und zu verbessern, die dazu verwendet werden, den Zustand eines Fahrzeugführers (12) zu bestimmen, wobei der Zustand des Fahrzeugführers (12) bestimmt wird, indem aus den Bilddaten ein Zustandssignal abgeleitet wird, das einem biologischen oder physiologischen Zustand des Fahrzeugführers (12) in Bezug auf biologische Messwerte zugeordnet ist, die die Herzfrequenz des Fahrzeugführers (12) und/oder die Atemfrequenz des Fahrzeugführers (12) umfassen, wobei das System Folgendes umfasst:
eine Kamera (302), die so positioniert ist, dass sie Bilddaten des Fahrzeugführers (12) erfasst;
Nicht-Bildsensoren (602) zum Bereitstellen von Nicht-Bildsensordaten bezüglich biologischer Messwerte des Fahrzeugführers (12);
ein Beleuchtungssystem (206, 310), das so positioniert ist, dass es einen Innenraum des Fahrzeugs beleuchtet;
eine Steuerung (600), die dafür konfiguriert ist, die erfassten Bilddaten zu empfangen und die Nicht-Bildsensordaten von den Nicht-Bildsensoren (602) zu empfangen; wobei:
die Steuerung (600) dafür konfiguriert ist, den Zustand des Fahrzeugführers (12) basierend auf den empfangenen Bilddaten zu bestimmen;
die Steuerung (600) dafür konfiguriert ist, die Qualität der Bilddaten zu bestimmen; und
wenn die Steuerung (600) bestimmt, dass die empfangenen Bilddaten eine schlechte Bildqualität aufweisen, so dass die biologischen Messwerte des Fahrzeugführers (12) nicht aus den empfangenen Bilddaten abgeleitet werden können, die Steuerung dafür konfiguriert ist, entweder das Beleuchtungssystem basierend auf der bestimmten Bildqualität zu ändern, um die Bildqualität zu verbessern, oder den Zustand des Fahrzeugführers (12) basierend sowohl auf den Bilddaten des Fahrzeugführers (12) als auch auf den Nicht-Bildsensordaten von den Nicht-Bildsensoren (602) zu bestimmen, indem die Bilddaten mit den Nicht-Bildsensordaten ergänzt werden.

2. System nach Anspruch 1, wobei die Steuerung (600) ferner dafür konfiguriert ist, basierend auf dem bestimmten Zustand des Fahrzeugführers (12) ein Ausgangssignal an eine Fahrzeugkomponente zu senden.

3. System nach Anspruch 2, wobei das Ausgangssignal das Anzeigen von Daten, die auf einer im Fahrzeuginneren angeordneten Anzeigevorrichtung dargestellt werden sollen; oder
das Ausgeben hörbarer Daten von einem Soundsystem veranlasst.

4. System nach Anspruch 1, wobei das Ändern des Fahrzeugbeleuchtungssystems (206, 310) das Erhöhen der Intensität von mindestens einer Lichtquelle in dem Beleuchtungssystem (206, 310) umfasst.

5. System nach Anspruch 1, wobei das Ändern des Fahrzeugbeleuchtungssystems (206, 310) das Einstellen der Frequenz der Bilddaten umfasst, die an der Kamera (302) erfasst werden.

6. System nach Anspruch 1, wobei die Nicht-Bildsensoren (602) einen Drucksensor, einen Wandler für ein elektrisches Feld, einen piezoelektrischen Sensor, einen Ultraschallwandler und/oder ein Mikrofon umfassen.

7. System nach Anspruch 1, wobei die Bilddatenqualität ein Maß des Signal-Rausch-Verhältnisses ist.

## Revendications

1. Système configuré pour recevoir et améliorer la qualité des données d'image utilisées pour déterminer l'état d'un conducteur de véhicule (12), l'état du conducteur de véhicule (12) étant déterminé en dérivant à partir des données d'image un signal d'état associé à l'état biologique ou physiologique du conducteur de véhicule (12) lié à des mesures biologiques comprenant au moins l'une parmi la fréquence cardiaque du conducteur de véhicule (12) et la fréquence respiratoire du conducteur de véhicule (12), le système comprenant :
une caméra (302) positionné pour détecter des données d'image du conducteur de véhicule (12) ; des capteurs sans image (602) pour fournir des données de capteur sans image concernant des mesures biologiques du conducteur de véhicule (12) ;
un système d'éclairage (206, 310) positionné pour éclairer un intérieur du véhicule ;
un contrôleur (600) configuré pour recevoir les données d'image détectées et pour recevoir les données de capteur sans image à partir des capteurs sans image (602) ; dans lequel :
le contrôleur (600) est configuré pour déterminer l'état du conducteur de véhicule (12) sur la base des données d'image reçues ;
le contrôleur (600) est configuré pour effectuer une détermination de la qualité des données d'image ; et
le contrôleur est configuré pour, si le contrôleur (600) détermine que les données d'image reçues ont une mauvaise qualité d'image de sorte que les mesures biologiques du conducteur de véhicule (12) ne peuvent pas être dérivées à partir des données d'image reçues, de manière sélective soit modifier le système d'éclairage sur la base de la qualité de données d'image déterminée pour améliorer la qualité des données d'image ou déterminer l'état du conducteur de véhicule (12) sur la base à la fois des données d'image du conducteur de véhicule (12) et des données de capteur sans image à partir des capteurs sans image (602) en complétant les données d'image avec les données de capteur sans image.

2. Système selon la revendication 1, dans lequel le contrôleur (600) est en outre configuré pour envoyer un signal de sortie à un composant du véhicule sur la base de la condition déterminée du conducteur de véhicule (12).

3. Système selon la revendication 2, dans lequel le signal de sortie incite l'une des : données d'affichage à être présentées sur un dispositif d'affichage situé dans l'habitacle du véhicule ; et des données sonores à être émises à partir d'un système de sonorisation.

4. Système selon la revendication 1, dans lequel la modification du système d'éclairage du véhicule (206, 310) comprend l'augmentation de l'intensité d'au moins une source lumineuse dans le système d'éclairage (206, 310).

5. Système selon la revendication 1, dans lequel la modification du système d'éclairage du véhicule (206, 310) comprend le réglage de la fréquence des données d'image détectées au niveau de la caméra (302).

6. Système selon la revendication 1, dans lequel les capteurs sans image (602) comprennent au moins l'un d'un capteur de pression, d'un transducteur de champ électrique, d'un capteur piézoélectrique, d'un transducteur à ultrasons et d'un microphone.

7. Système selon la revendication 1, dans lequel la qualité des données d'image est une mesure du rapport signal sur bruit.
